# EUROPEAN PATENT SPECIFICATION

(11) **EP 2 306 993 B1**
(45) Date of publication and mention of the grant of the patent: **12.03.2014**
(21) Application number: 09753885.4
(22) Date of filing: 26.05.2009
(51) Int. Cl.: A61K 9/51, A61K 47/48

(54) **PROTAMINE/RNA NANOPARTICLES FOR IMMUNOSTIMULATION**
PROTAMIN/RNA-NANOPARTIKEL FÜR DIE IMMUNSTIMULIERUNG
NANOPARTICULES D'ARN/PROTAMINE POUR L'IMMUNOSTIMULATION

(30) Priority: 26.05.2008 EP 08009581
(43) Date of publication of application: 13.04.2011
(73) Proprietor: Universität Zürich, 8006 Zürich (CH)
(72) Inventor: PASCOLO, Steve, CH-8004 Zürich (CH); KNUTH, Alexander, CH-8002 Zürich (CH)
(74) Representative: Habermann, Hruschka & Schnabel
(86) International application number: PCT/EP2009/056391
(87) International publication number: WO 2009/144230

(56) References cited:
- WO-A-03/051401
- WO-A-2005/016376
- WO-A-2007/121347
- WEYERMANN J ET AL: "Albumin-protamine-oligonucleotide-nanopar ticles as a new antisense delivery system. Part 2: cellular uptake and effect" EUROPEAN JOURNAL OF PHARMACEUTICS AND BIOPHARMACEUTICS, ELSEVIER SCIENCE PUBLISHERS B.V., AMSTERDAM, NL, vol. 59, no. 3, 1 April 2005 (2005-04-01), pages 431-438, XP025317611 ISSN: 0939-6411 [retrieved on 2005-04-01]
- HOERR I ET AL: "In vivo application of RNA leads to induction of specific cytotoxic T lymphocytes and antibodies" EUROPEAN JOURNAL OF IMMUNOLOGY, WILEY - V C H VERLAG GMBH & CO. KGAA, DE, vol. 30, no. 1, 1 January 2000 (2000-01-01), pages 1-7, XP002243972 ISSN: 0014-2980
- LI SHYH-DAR ET AL: "Targeted delivery of antisense oligodeoxynucleotide and small interference RNA into lung cancer cells" MOLECULAR PHARMACEUTICS, AMERICAN CHEMICAL SOCIETY, US, vol. 3, no. 5, 1 October 2006 (2006-10-01), pages 579-588, XP009121365 ISSN: 1543-8384

### SEQUENCE LISTING

<110> universität Zürich
<120> Protamine/RNA nanoparticles for immunostimulation
<130> U 0016 WOEP
<140> EP 09753885.4
   <141> 2009-05-26
<150> EP 08009581.3
   <151> 2008-05-26
<160> 13
<170> PatentIn version 3.3
<210> 1
   <211> 18
   <212> RNA
   <213> Artificial
<220>
   <223> U rich oligonucleotide RNA18UR
<400> 1
   aguguuauuc uuguaugg 18
<210> 2
   <211> 21
   <212> RNA
   <213> Artificial
<220>
   <223> oligonucleotide oligo 21
<400> 2
   guugcauggg ccucauauac a 21
<210> 3
   <211> 18
   <212> RNA
   <213> Artificial
<220>
   <223> oligonucleotide RNA18UP
<400> 3
   aucgcuaucg aucucuag 18
<210> 4
   <211> 12
   <212> RNA
   <213> Artificial
<220>
   <223> oligonucleotide A-rich 12m
<400> 4
   gaaaaacaag aa 12
<210> 5
   <211> 12
   <212> RNA
   <213> Artificial
<220>
   <223> oligonucleotide u-rich 12m
<400> 5
   guuauucuug ua 12
<210> 6
   <211> 12
   <212> RNA
   <213> Artificial
<220>
   <223> oligonucleotide C-rich 12m
<400> 6
   gccacccccg ca 12
<210> 7
   <211> 12
   <212> RNA
   <213> Artificial
<220>
   <223> Oligonucleotide G-rich 12m
<400> 7
   gggaggcgga ga 12
<210> 8
   <211> 12
   <212> RNA
   <213> Artificial
<220>
   <223> oligonucleotide Purine 12m
<400> 8
   agagagagag ag 12
<210> 9
   <211> 12
   <212> RNA
   <213> Artificial
<220>
   <223> Oligonucleotide Pyrimidine 12m
<400> 9
   cucucucucu cu 12
<210> 10
   <211> 21
   <212> RNA
   <213> Artificial
<220>
   <223> oligonucleotide 12merU
<400> 10
   aguguuauuc uuguaugguu g 21
<210> 11
   <211> 16
   <212> RNA
   <213> Artificial
<220>
   <223> oligonucleotide 16merU
<400> 11
   guguuauucu uguaug 16
<210> 12
   <211> 14
   <212> RNA
   <213> Artificial
<220>
   <223> oligonucleotide 14merU
<400> 12
   guuauucuug uaug 14
<210> 13
   <211> 10
   <212> RNA
   <213> Artificial
<220>
   <223> oligonucleotide 10meru
<400> 13
   guuauucuug 10

## Description

The present invention relates to immunostimulating protamine/RNA nanoparticles of defined average size, a pharmaceutical composition containing said nanoparticles and to a method of producing the same. The nanoparticles of the present invention are particularly useful as immunomodulating medicament with a precise pattern of immunostimulation different from the prior art.

### Background of the Invention

In addition to their central role as carriers of genetic information, certain nucleic acid molecules have recently been recognized to be pathogen-associated molecular patterns (PAMP) and have been used as agents for stimulating host innate immune responses. These immunostimulatory nucleic acid molecules include deoxynucleotides (DNA) comprising the unmethylated CpG motif (CpG oligodeoxynucleotide: CpG ODN) and ribonucleotides (RNA) in the form of doublestranded RNAs, and protected or otherwise chemically modified single-stranded RNA molecules (ssRNAs). The different families of nucleic acid PAMPs (i.e. CpG ODN, dsRNA, and. stabilized ssRNAs) are known to trigger different Toll-like receptors (TLRs) expressed by non-overlapping cell populations (Jarrossay wt al., 2001, Specialization and complementarity in microbial molecule recognition by human myeloid and plasmacytoid dendritic cells. Eur. J Immunol. 31: 3388-3393), and give rise to different types of innate immune responses characterized by the secretion of a specific panel of cytokines.

DNA as adjuvant or as vaccine delivery vehicle, however, may cause safety concerns, for example, due to the risk of the DNA integrating into the cellular genome, causing mutations which deregulate gene expression (induce or impede a gene expression). Such mutations may also be transforming, posing a serious risk for causing cancer in the patient.

Furthermore, CpG ODN, the synthetic version of bacteria DNA for immunostimulation, or bacterial DNA may trigger severe side effects due to overstimulation of the immune system (see e.g. Heikenwalder et al., (2004) Nat. Med. 10:187-192) which is known to result in splenomegaly in mice (Montheith et al., Anticancer Drug Res. 1997, 12 (5): 421-432). Moreover, DNA molecules are degraded relatively slowly in the bloodstream, and as such may cause the formation of auto-antibodies (Gilkeson et al., J. Clin. Invest. 1995, 95: 1398-1402).

Recently it was demonstrated that stabilized RNA in the form of messenger RNA (RNA) condensed on the natural cationic peptide protamine or synthetic oligoribonucleotides (ORN) with a phosphorothioate backbone are danger signals for mouse antigen-presenting cells (APC) (see Scheel et al., Immunostimulating capacities of stabilized RNA molecules. Eur. J. Immunol. 2004. 34: 537-547, EP1806139 B1 for chemically modified RNA oligonucleotides and Rep1818409 B1 for protamine-protected mRNA)).

In other reports instead of protamine, cationic lipids or polyethylenimine (PEI) are used to protect and transfect the RNA. Heil et al., Species-specific recognition of single-stranded RNA via Toll-like receptor 7 and 8. Science 2004. 303: 1526-1529, and WO 03/086280 A2, identified mouse TLR-7 and human TLR-8 as the receptors for phosphorothioate ORN delivered via liposomes. Specifically, it was reported that guanosine (G)- and uridine (U)-rich ssRNA oligonucleotides derived from HIV-1 stimulated dendritic cells in macrophages to secrete interferon-α, recognizing that GU-rich ssRNA oligonucleotides (ORN) are a class of physiological ligands for TLR-7 and TLR-8. Diebold et al. (2004, Innate antiviral responses by means of TLR7-mediated recognition of single-stranded RNA. Science 303:1529-1531) reported that ssRNAs protected by PEI also induce TLR7-dependent production of inflammatory cytokines, and postulated that cells of the innate immune system sense endosomal ssRNAs to detect infection by RNA viruses. The same authors further demonstrated that PEI-delivered RNA oligonucleotides stimulate preliminary through their U residues. Meanwhile, Hornung et al (Nat Med. 2005 Mar; 11 (3):263-70 and EP 1 764 107 A1) tested many RNA oligonucleotides delivered by cationic liposomes and could from these results develop an algorithm that can predict the immunostimulatory capacity of RNA sequences. Lund et al., Recognition of single-stranded RNA viruses by Toll-like receptor 7. Proc. Natl. Acad. Sci. USA 2004. 101: 5598-5603, showed that the single-stranded RNA genomes of viruses (influenza virus, vesicular stomatitis virus) are recognized by TLR-7 in mice. Finally, Kariko et al., mRNA is an endogenous ligand for Toll-like receptor 3. J. Biol. Chem, 2004. 279: 12542-12550, demonstrated that mRNA stabilized by encapsulation in liposomes or by 2' fluoro modifications activates DC through TLR-3. Because stabilized ssRNA activates the innate immunity, it can be used as an adjuvant for vaccines as described by Scheel et al. for naked phosphorothioate RNA oligonucleotides (Scheel, European Journal of Immunology, 2004) and Bourquin (Bourquin, Blood, 1 April 2007, Vol. 109, No. 7, pp. 2953-2960) for liposome encapsulated RNA oligonucleotides.

Diebold et al., (Innate antiviral responses by means of TLR7-mediated recognition of single stranded RNA. Science 2004. 303: 1529-1531) showed that RNA molecules protected by PEI stimulate mouse plasmacytoid DC (pDC) through TLR-7.

More recently, Diebold et al. (2006) (Eur. J. Immunol. 36:3256-3267) reported that uridine and ribose are both necessary and sufficient for TLR7 stimulation, and that ssRNAs act as TLR7 agonists in a sequence-independent manner as long as they contain several uridines in close proximity. Dielbold et al. (2006) further showed that, when delivered to endosomes, viral and self RNA triggered equally efficiently TLR7 mediated innate immune response, further supporting the notion that discrimination between self and viral RNA ligands is based on endosomal accessibility rather than RNA sequence. It was also recently disclosed that mRNAs transcribed from the E. coli β-galactosidase or CMV pp65 gene, when complexed with protamine, can induce therapeutic anti-tumor immunity in tumor bearing mice (Scheel et al. ,2006, Eur. J. Immunol. 36:2807-2816).

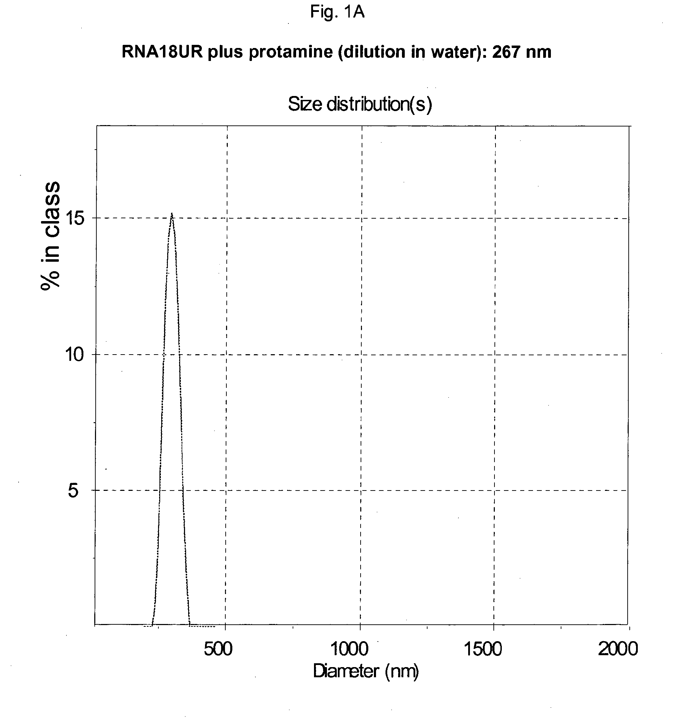

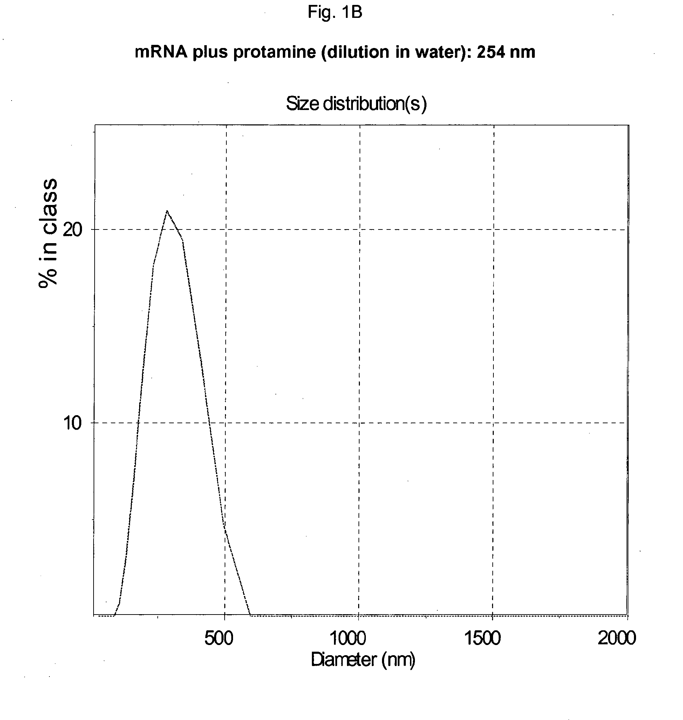

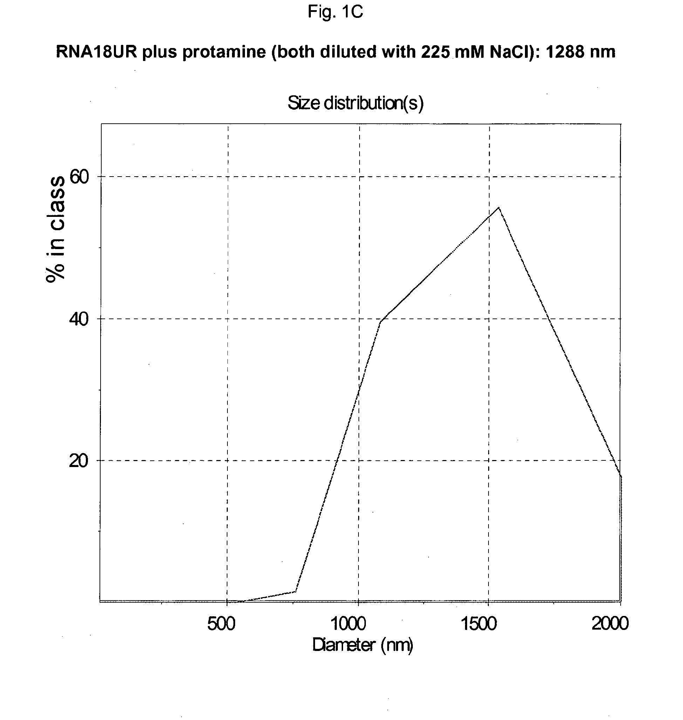

None of the above RNA-based immunostimulating compositions, however, are optimal for clinical applications because they require formulations with cationic liposomes which are unstable in vitro and toxic or with PEI which is toxic (review by Hunter AC. Molecular hurdles in polyfectin design and mechanistic background to polycation induced cytotoxicity. Adv Drug Deliv Rev. 2006 Dec 1;58(14):1523-31) or with protamine that gives heterogeneous large aggregates (Scheel et al. (2005), Eur. J. Immunol. 35: 1557-1566, see Fig. 1 thereof) which quickly precipitate and are not easily taken up in a syringe and injectable (they pellet in the tube or get trapped in the syringe or the needle). Moreover, most of these formulations are made with modified RNA oligonucleotides which are foreign to the body and may trigger an anti-vaccine immune response, i.e. an antibody response specific for the chemical modifications in the oligonucleotide. Therefore, there remains a need for a clinically acceptable immunostimulating formulation based on unmodified RNA as Active Pharmaceutical Ingredient. It is the object of the present invention to provide methods and compositions to overcome the above shortcomings of the prior art. Because it is based on homogenous, well-defined nanoparticles, the present "immunostimulatory protamine-RNA nanoparticle composition" is suitable for pharmaceutical formulation and any type of injection, including intra-venous.

On the other hand, nucleic acid based immuno-stimulants are known to increase, in addition to the level of desirable IFN-alpha which is one of the very few cytokine used as a drug (treatment of hepatitis C-virus infections), the level of inflammatory cytokines, such as TNF-alpha (see e.g. U.S. Pat. No. 6,429,199), which is known to be toxic and dose limiting.

TNF-alpha is a cytokine released primarily by cells of the immune system in response to certain immunostimulators. When administered to animals or humans, it causes inflammation, fever, cardiovascular effects, hemorrhage, coagulation, cachexia, and acute phrase responses similar to those seen during acute infections, inflammatory diseases, and shock states. Excessive or unregulated TNF-alpha production has been implicated in a number of disease conditions. These include endotoxemia and/or toxic shock syndrome [(Tracey, et al., (1990) Nature 330, 662-664 (1987) and Hinshaw, et al., Circ. Shock 30, 279-292)], rheumatoid arthritis, inflammatory bowel disease, cachexia [(Dezube, et al., (1990) Lancet, 335 (8690), 662], and lupus. Systemic infusion of recombinant TNFα resulted in changes typically seen in adult respiratory distress syndrome [(Ferrai-Baliviera, et al., (1989) Arch. Surg. 124(12), 1400-1405)]. TNF-alpha has pro-inflammatory activities which together with its early production (during the initial stage of an inflammatory event) make it a likely mediator of tissue injury in several important disorders including but not limited to, myocardial IFN-alpharction, stroke and circulatory shock.

It has also been reported that TNF-alpha is a potent activator of retrovirus replication including activation of HIV-1. [(Duh, et al., Proc. Nat. Acad. Sci. 86, 5974-5978 (1989); Poll, et al., Proc. Nat. Acad. Sci. 87, 782-785 (1990); Monto, et al., Blood 79, 2670 (1990); Clouse, et al., J. Immunol. 142, 431-438 (1989); Poll, et al., AIDS Res. Hum. Retrovirus, 191-197 (1992)].

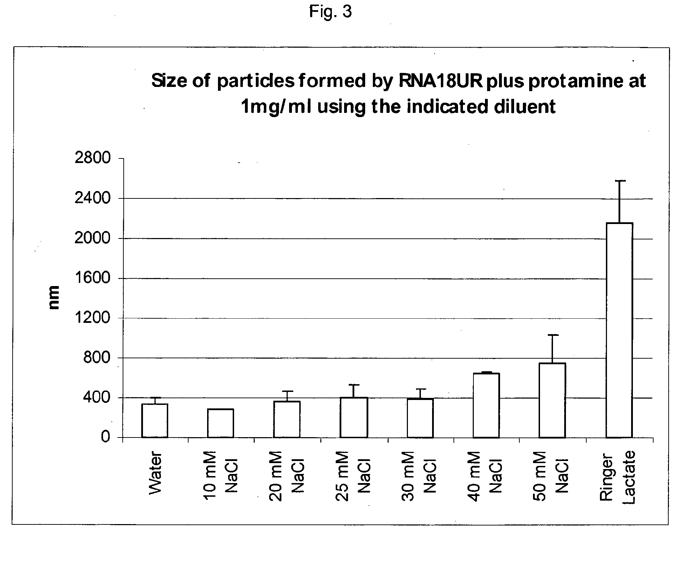

Scheel et al. (2005; see Fig. 3A) published that large aggregates made by mixing protamine and RNA under prior art conditions (i.e. in the presence of salts), induce a strong production of TNF-alpha by human immune cells.

WO2005016376, WO03051401, WO2007121347, Weyermann et al. European Journal of Pharmaceuticics and Biopharmaceutics. 59, 3. 2005. 431-438, Hoerr et al. European Journal of Immunology. 30, 1. 2000. 1-7 and Li Shyh-Dar et al. Molecular Pharmaceutics. 3, 5. 2006. 579-588 disclose RNA compositions wherein protamine may be included and corresponding uses.

In view of the shortcomings of prior art approaches it would be highly desirable to have an immunostimulatory composition that could be custom made to induce production of IFN-alpha/TNF-alpha in a controlled fashion, in particular to induce the production of IFN-alpha yet without strongly increasing the level of TNF-alpha.

The solution to the above technical problem is provided by the embodiments of the present invention as defined in the claims.

### Summary of the Invention

In particular, according to a first aspect the present invention provides nanoparticles of an average size of from 50 to 450 nm comprising protamine and RNA. The nanoparticles of the present invention are particularly for use as an immunostimulating medicament.

Thus, according to a second aspect, the present invention provides a pharmaceutical composition comprising such nanoparticles of protamine and RNA in combination with a pharmaceutically acceptable excipient, vehicle and/or diluent.

According to a third aspect, the present invention is directed to a method for the production of the nanoparticles according to claim 12, which method comprises the steps of:
(a) providing an aqueous solution of RNA
   (1) at 1 mg/ml or more using a solution of 0 to 75 mM electrolytes; or
   (2) at less than 1 mg/ml using a solution of 0 to 225 mM electrolytes;
(b) providing an aqueous solution of protamine
   (1) at 1 mg/ml or more using a solution of 0 to 75 mM electrolytes;or
   (2) at less than 1 mg/ml using a solution of 0 to 225 mM electrolytes; and
(c) combining the solutions obtained in steps (a1) and (b1) or mixing the solutions obtained in (a2) and (b2).

Preferably, the above steps (a1 and/or a2) are performed by resuspending an appropriate amount of dried RNA either (a1) in an aqueous solution containing 0 to 75 mM electrolytes or (a2) in an aqueous solution containing 0 to 225 mM electrolytes.

Preferably, above steps (b1) and/or (b2) are carried out by diluting an aqueous isotonic stock solution of protamine, preferably containing 1000 ("protamine 1000") to 5000 ("protamine 5000") heparin-neutralizing units per ml with a solution containing either (1) 0 to 75 mM salts or (2) 0 to 225 mM salts. For example, protamine 1000 and 5000 stock solutions are commercially available from Valeant Pharmaceuticals International, Aliso Viejo, CA, USA, under the trademarks Valeant^{®} 1000 and 5000, respectively.

Most preferred, the method according to the present invention comprises the following steps:
(a) providing an aqueous solution of RNA at less than 2 mg/ml in pure water;
(b) providing an aqueous solution of protamine at less than 2 mg/ml by diluting an aqueous isotonic stock solution containing 5000 heparin-neutralizing units of protamine per ml with pure water; and
(c) combining the solutions obtained in steps (a) and (b).

In this context, it should be noted that, once the nanoparticles of the present invention are formed, the specific salt (or electrolyte)/concentration conditions for RNA and protamine according to the method of the present invention need not to be further maintained. Thus, the nanoparticles can be further processed, e.g. eventually recovered by centrifugation and diluted, dissolved or dispersed in a medium, preferably a pharmaceutically acceptable excipient, vehicle and/or diluent, in particular in an isotonic medium such as Ringer or Ringer Lactate solution.

The nanoparticles according to the invention are particularly useful as an immunomodulating, preferably immunostimulatory medicament. The present invention therefore also relates to a method for stimulating the immune system of mammalian patient comprising the administration of an effective amount of a pharmaceutical composition of the present invention.

The present invention is based, at least in part, on the discovery that compositions of protamine and RNA can be provided as nanoparticles of defined size by diluting RNA (preferably oligonucleotides or mRNA) and protamine in water or aqueous solutions having low ionic strength (preferably less than about 75 mM salts, more preferred less than 25 mM salts) and mixing the two solutions (see Figs. 1 to 3 and Table 1). This surprising finding is contrary to the prior art wherein the presence of higher electrolyte concentrations in RNA and/or protamine resulted in the production of large particles and aggregates.

According to the present invention, the term "mM salts" means the concentration in 10⁻³ mol per liter of the sum of all electrolytes (including inorganic salts such as Nacl, KCI, NaH₂PO₄, Na₂HPO₄, KH₂PO₄, K₂HPO₄, MgCl₂, MnCl₂, Na₂SO₄, K₂SO₄, MgSO₄ and salts such Tris-HCl, EDTA, Hepes, etc.) in the solutions used to resuspend or to dilute an RNA stock solution and in the solutions used to dilute a Protamine stock solution (such as Protamine 1000 or 5000) before mixing the components (i.e. (a1) and (b1) or (a2) and (b2) as defined above).

In the context of the present invention the terms "salt(s)" and "electrolyte(s)" are used interchangeably and mean a compound that at least partially dissociates into its respective counter ions in water.

According to a preferred embodiment of the present invention, nanoparticles of protamine and RNA can be prepared by diluting both RNA and protamine (preferably from a stock solution such as protamine 5000) to less than 2 mg/ml, preferably 1 mg/ml in pure water to have an average size below 500 nm, in particular 50 to 450 nm. Such nanoparticles can be used for immunostimulation (or for the preparation of a medicament for immunostimulation) by inducing strongly IFN-alpha but TNF-alpha to a lesser extent (see Figs. 4 and 5). Furthermore, such small-sized particles have the additional benefit to be injectible, e.g. by intra venous injection (cf. Fig. 6).

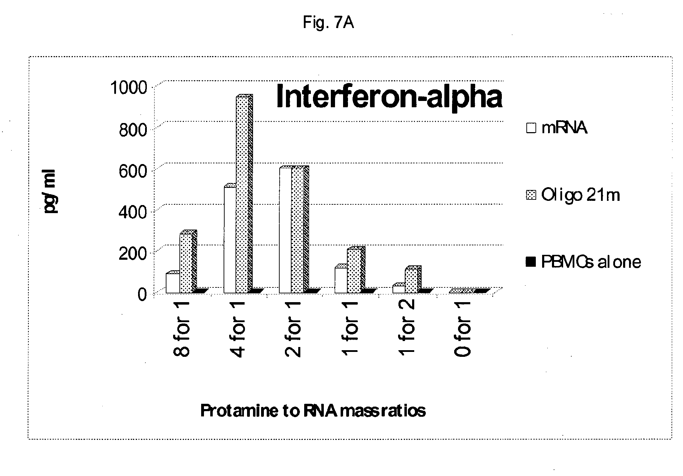

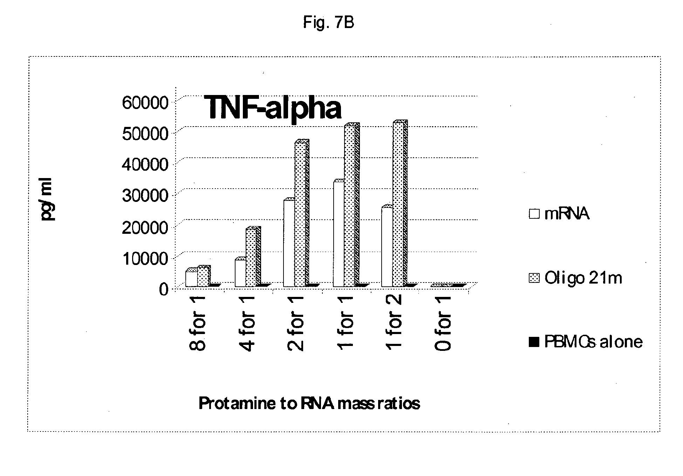

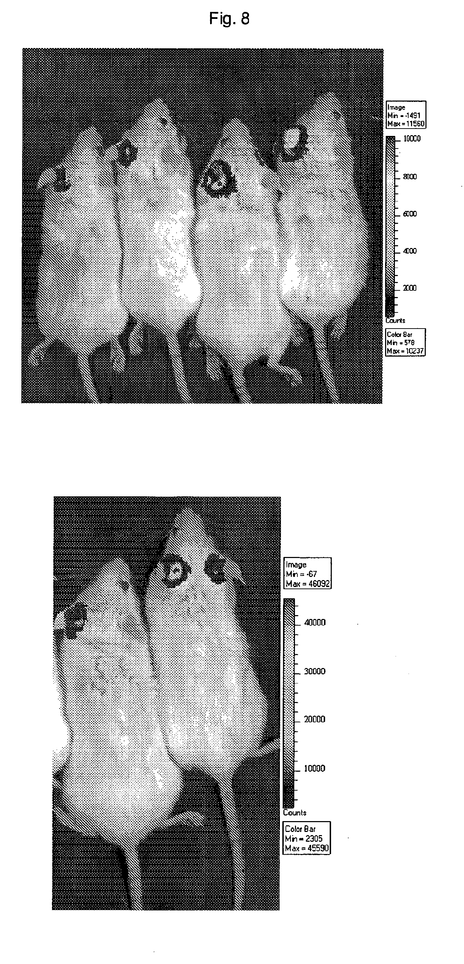

According to a further preferred embodiment of the invention a mass ratio of protamine to RNA of 1 to 1 or higher (i.e. more protamine than RNA) results in an optimal immunostimulation (Fig. 7) while a ratio of 1 to 4 (i.e. four times less protamine than RNA) or lower results in mRNA expression (Fig. 8).

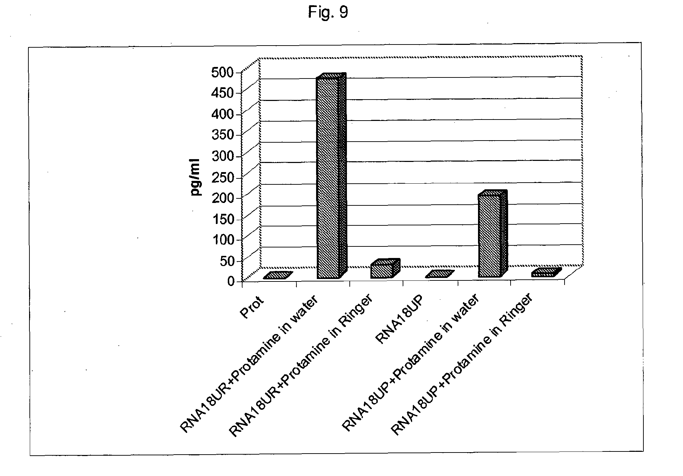

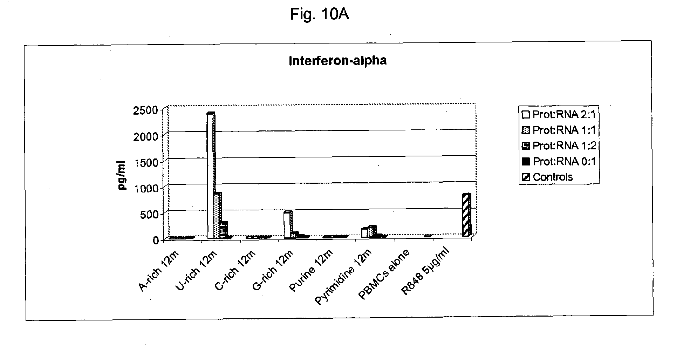

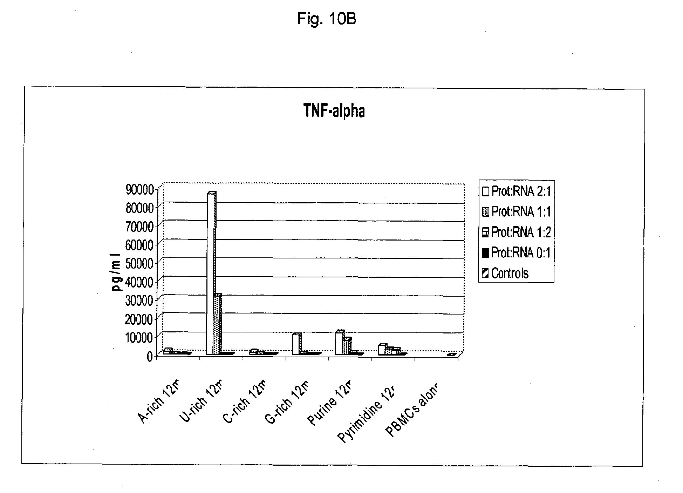

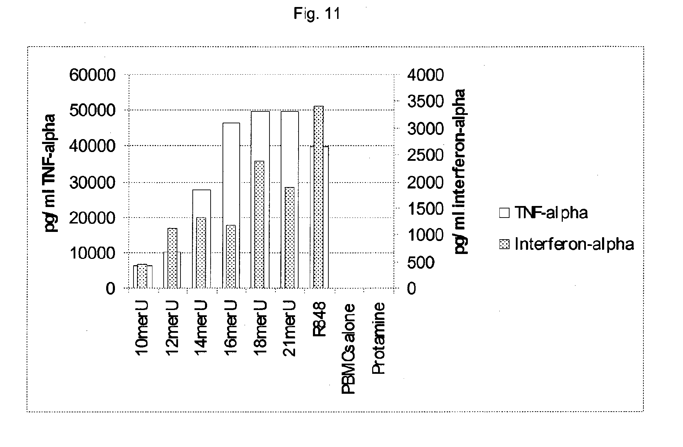

According to the present invention the presence of a double U or UG doublet in the RNA is not necessary for protamine-RNA complexes to stimulate IFN-alpha production by PBMCs; cf. Fig. 9. However, an oligonucleotide containing at least one U residue is usually preferred (see Fig. 10). Preferred oligoribonucleotides of the present invention contain 6 to 100 nucleotides. For example, oligonucleotides of 10 residues complexed with protamine are shown to stimulate immune cells. Stimulation of human PBMCs is shown to be optimal with RNA oligonucleotides of 16 or more residues (see Fig. 11).

Accordingly, in one embodiment, (i) protamine formulated as a 1 mg/ml solution thanks to the dilution with pure water of a pharmaceutical isotonic solution of at least 10 mg/ml (protamine 5000) and (ii) RNA formulated as a 1 mg/ml solution by dilution of a dried RNA pellet in pure water, when mixed together, form homogenous nanoparticles of approx. 250 nm in diameter in average.

The benefits of the present invention are independent of the RNA length (it works equally well with oligoribonucleotides of a minimum of about 6 bases and with long in vitro transcribed mRNA). When electrolytes are present in the solutions used to dilute RNA or protamine before mixing (e.g. dilution of protamine and RNA to 1 mg/ml using isotonic solutions such as Ringer Lactate), the particles obtained are larger (around 25 µm) and heterogenous in size, as shown earlier (Scheel et al, 2005, Fig. 1).

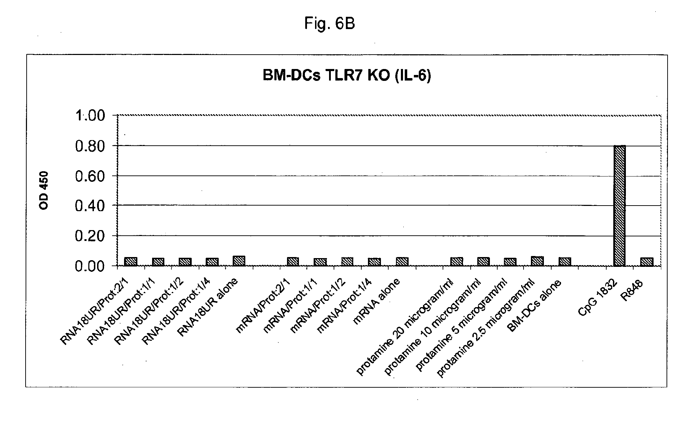

It was surprisingly found that the smaller particles obtained according to the method of the invention (preferably by mixing protamine 5000 diluted 14 times in pure water and RNA at 1 mg/ml in pure water) induce strongly the production of IFN-alpha but comparatively only weakly the production of TNF-alpha, whereas the larger particles induce strongly both cytokines (Fig. 6B). In further preferred embodiments, the RNA comprises more than 10 nucleotides and contains one or more U residues. However, no double U residues are needed. G residues can also induce some level of immunostimulation. However, contrary to previous reports (see WO-A-2007/031322, WO-A-2008/014979) UG pairs or GU pairs are not required. The RNA of the present invention does not need to have the features of a messenger RNA (more than 100 residues, start codon, stop codon, poly-A tail) as previously suggested in the prior art (Scheel, 2004, Scheel 2005 and Scheel 2006).

### Brief Description of the Drawings

Other objects, advantages and novel features of the present invention will become apparent from the following detailed description when considered in conjunction with the accompanying drawings.

Fig. 1 shows examples of raw data obtained from size measurement experiments for different protamine/RNA compositions. The 18 mer U rich RNA oligonucleotide (RNA18UR: 5' AGUGUUAUUCUUGUAUGG 3', (SEQ ID NO: 1); stock solution at 5 mg/ml in water) or messenger RNA (mRNA, approx. 900 bases, 5 mg/ml stock solution in water) and protamine VALEANT^{®} 5000 (14 mg/ml stock solution in an isotonic injection solution) were formulated at 2 mg/ml, 1 mg/ml, 0.5 mg/ml or 0.25 mg/ml in pure water or in water plus 25 mM NaCl or in water plus 75 mM NaCl or in water plus 225 mM NaCl or in Ringer Lactate. Equal amounts of RNA and protamine were mixed together (5 microliters of each solution). 1 minute later (after particles are formed), 1 ml of Ringer lactate was added and the whole solution transferred to a transparent cuvette. The cuvette is placed in a Malvern Zetasizer and the size of the particles was measured. It is demonstrated that RNA diluted to 1 mg/ml in water mixed with protamine 5000 diluted to 1 mg/ml in water when mixed together generate nanoparticles with an average size (diameter) of less than 500 nm (RNA18UR plus protamine (dilution in water): 267 nm; and mRNA plus protamine (dilution in water): 254 nm). On the contrary, when both RNA and protamine are diluted to 1 mg/ml in 225 mM NaCl solution, the complexes which are formed are larger and heterogenous (RNA18UR plus protamine, both diluted with 225 mM NaCl aqueous solution: 1288 nm). In the case of long RNA molecules (e.g. mRNA), the presence of higher electrolyte concentrations (e.g. dilution of RNA and protamine to 1 mg/ml with Ringer Lactate) induces the formation of large aggregates that cannot be detected by the Zetasizer (size of particles is out of the detectable range) but can be visualized with a standard microscope (see Scheel et al. 2005; Fig. 1).

Table 1 below summarizes the average particle-size (diameter) results obtained using RNA18UR and Protamine 5000 both diluted to 2 mg/ml or 1 mg/ml or 0.5 mg/ml or 0.25 mg/ml or 0.125 mg/ml) in pure water or 25 mM NaCl or 75 mM NaCl or 225 mM NaCl or Ringer Lactate (Ringer). It shows that electrolytes contained in the diluting solutions or coming from low dilution of protamine 5000 (when used at 2 mg/ml thus diluted only 7 times in water) dictate the formation of larger particles (more than 450 nm in diameter in average) than when RNA and protamine 5000 are both diluted to 1 mg/ml in water. To further dilute both RNA and protamine to less than 1 mg/ml is a way to reduce particle sizes even when, e.g. a 25 mM or 75 mM or 225 mM NaCl or Ringer Lactate solution is used.

**Table 1:**

| | | | | | |
|---|---|---|---|---|---|
| Concentration of RNA and Protamine [mg/ml] | 2.0 | 1.0 | 0.5 | 0.25 | 0.125 |

| | Particle size [nm] | Particle size [nm] | Particle size [nm] | Particle size [nm] | Particle size [nm] |
|---|---|---|---|---|---|
| Water | 575 | 267 | 274 | 263 | |
| Water plus 25 mM NaCl | | 492 | | 277 | |
| Water plus 75 mM NaCl | | 1045 | | 512 | |
| Water plus 225 mM NaCl | | 1288 | | 869 | |
| Ringer | 1363 | 1432 | 926 | 571 | 560 |

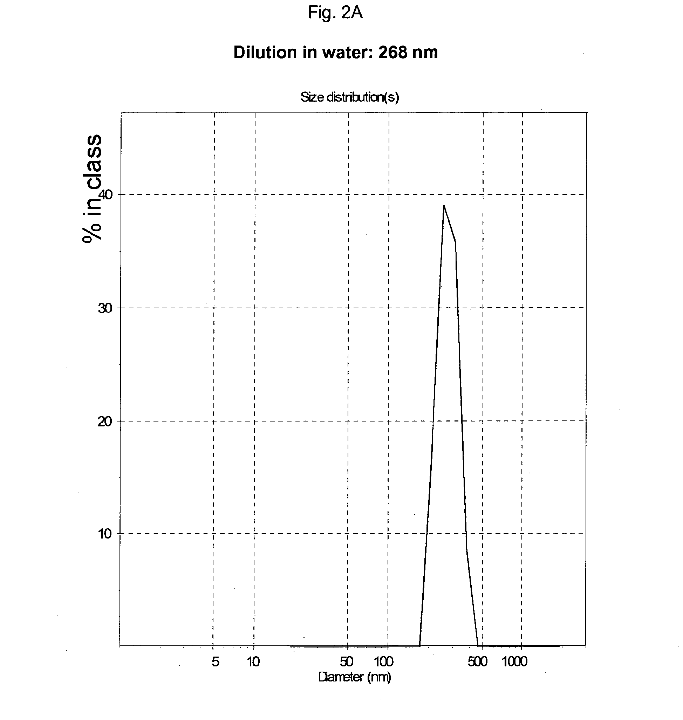

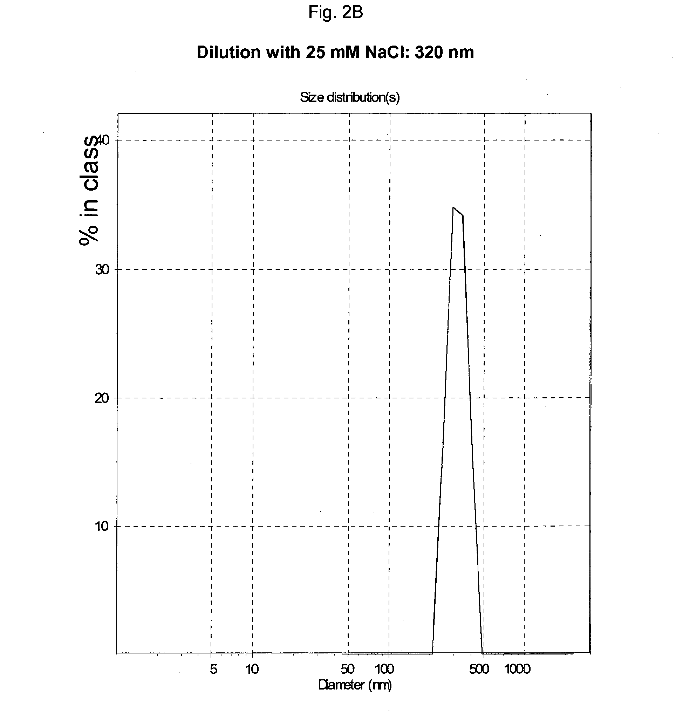

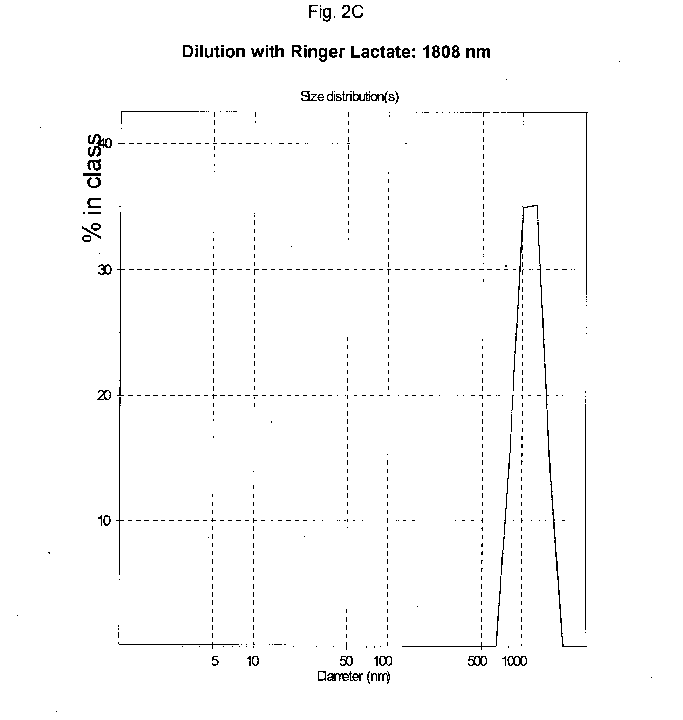

Fig. 2 shows the raw data of a particle size measurement experiment where RNA18UR and protamine are diluted to 1 mg/ml using water (dilution in water: 268 nm), or in 25 mM NaCl (dilution in 25 mM NaCl: 320 nm) or in Ringer Lactate (dilution in Ringer : 1808 nm). Similarly to the results shown in Fig. 1 and Table 1, the presence of increasing salts concentrations resulted in the production of protamine-RNA particles of increasing average sizes.

Fig. 3 summarizes the results of a further particle size measurement experiment in which RNA18UR and Protamine 5000 are diluted to 1 mg/ml with water or 10 mM NaCl or 20 mM NaCl or 25 mM NaCl or 30 mM NaCl or 40 mM NaCl or 50 mM NaCl or Ringer Lactate. At least two formulations and measurements were carried out for each condition.

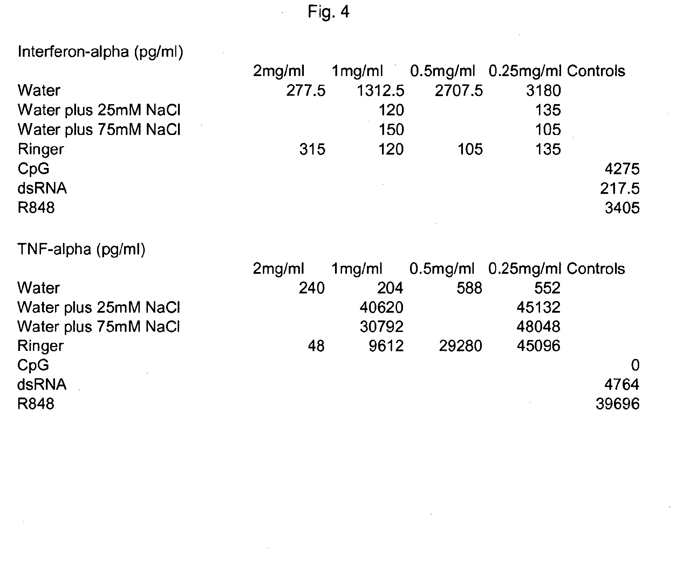

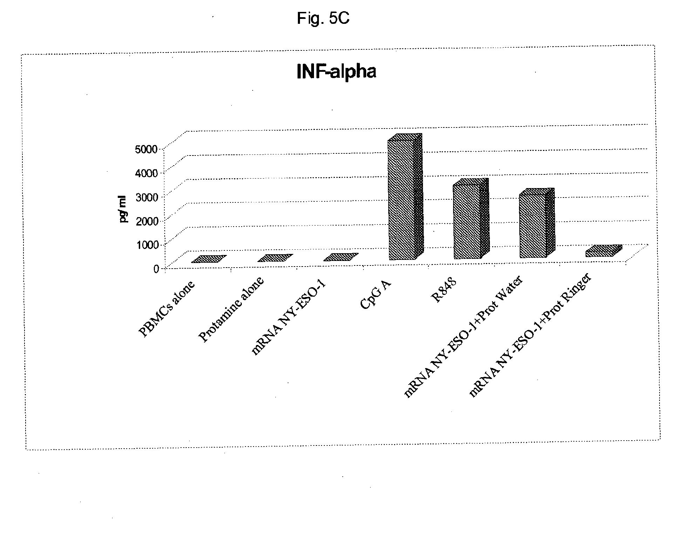

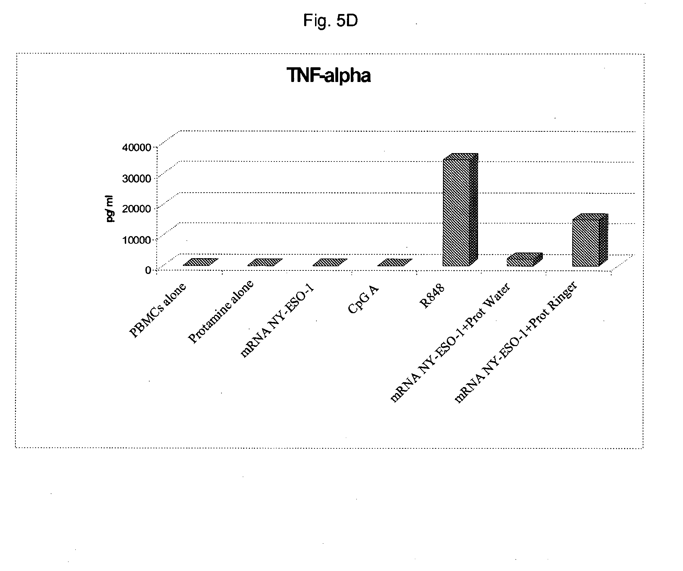

Fig. 4 shows the immunostimulation capacity of particles formed using the conditions of Table 1. PBMCs from healthy humans were prepared using Ficoll® gradient separation. They were then washed with PBS and resuspended at 5 million per ml in RPMI with 10% fetal calf serum plus penicillin and streptomycin. Two hundred microliters (1 million of cells) were added to the particles formed in the well of a 96 well plate by mixing 2 micrograms of RNA18UR with 2 micrograms of protamine 5000 both at the indicated concentration in the indicated solution. Positive controls are PBMCs similarly incubated with the phosphorothioate CpG class A DNA oligonucleotide, double stranded RNA (dsRNA) both at a final concentration of 10 microgram/ml or TLR7/8 agonist R848 at 2 microgram/ml final. These preparations were incubated for 24 hours at 37°C with 5% CO2. Then, the supernatants of the culture were collected and frozen at -20°C. The contents of IFN-alpha and TNF-alpha in these supernatants were evaluated using 20 microliters of thawn supernatants and ELISA kits from Bender (IFN-alpha) and Abazyme (TNF-alpha). The results are presented in pg/ml in the cell culture supernatant. They demonstrate that only particles generated by RNA18UR and protamine 5000 both diluted in water to 1 mg/ml or less (down to 0.25 mg/ml) stimulate a strong IFN-alpha production and relatively weak TNF-alpha production. The higher the dilution, the more IFN-alpha is produced (compare the results using 1 mg/ml and 0.25 mg/ml). The same results are obtained when instead of RNA18UR at 1 mg/ml, other RNA including mRNA in water at 1 mg/ml is used (Figs. 5C and 5D). When salts are present in RNA or Protamine-diluting solutions, TNF-alpha production is increased: only less than 600 pg/ml are obtained when water is used for dilution of protamine and RNA while more than 40000 pg/ml are obtained when 25 mM NaCl is used for the dilution.

It is known that CpG class A oligonucleotides induces the production of IFN-alpha but not of TNF-alpha. The TLR7/8 agonist R848 induces a high production of IFN-alpha and TNF-alpha. Double stranded RNA that stimulates immune cells through TLR3 induces a weak production of IFN-alpha and TNF-alpha by human PBMCs.

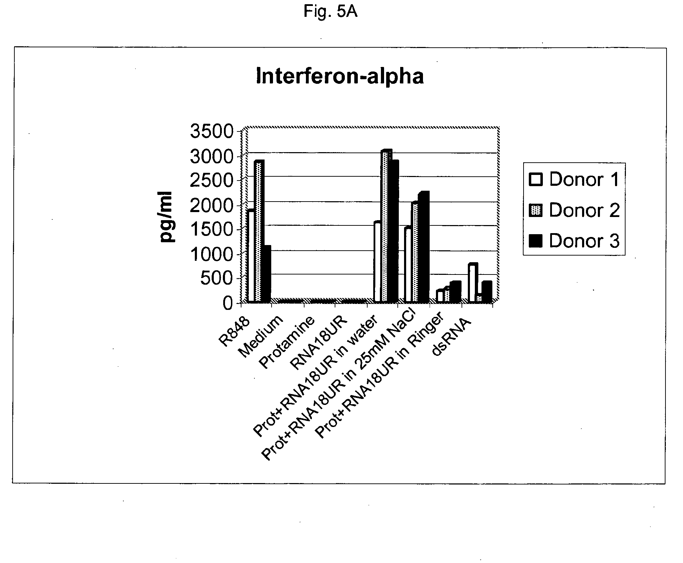

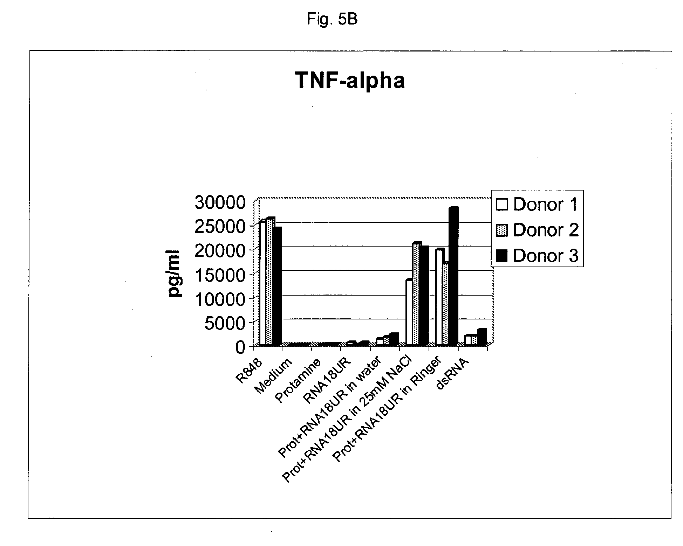

Fig. 5A and B: An independent experiment similar to the one shown in Fig. 4 was performed using fresh PBMCs from three healthy donors. RNA18UR and protamine 5000 were diluted to 1 mg/ml with water or with water containing 25 mM NaCl or with Ringer Lactate. As in Fig. 4, 200 microliters of fresh PBMCs at 5 million cells/ml were cocultured with 4 micrograms of particles (2 micrograms of RNA mixed with 2 micrograms of Protamine) for 24 hours at 37°C. IFN-alpha and TNF-alpha were then measured in the culture supernatant using ELISA kits. Whoever is the donor, when protamine 5000 and RNA are diluted with pure water to 1 mg/ml they form particles that induce high IFN-alpha (Fig. 5A) but low TNF-alpha (Fig. 5B). When protamine and RNA are diluted with 25 mM NaCl, in this experiment both high IFN-alpha and high TNF-alpha are induced. This is the case in most experiments. With larger particles produced by diluting Protamine 5000 and RNA in Ringer Lactate, high TNF-alpha but low IFN-alpha production is detected.

Fig. 5C and D: A further experiment was performed in order to evaluate whether mRNA/protamine nanoparticles according to the present invention exert immunostimulation. Approx. one million of human PBMCs at 5 million cells per ml (200 microliters) were cultured for 24 hours "alone" or with 2 micrograms (2 microliters) of "protamine alone" (protamine 5000 diluted to 1 mg/ml using water) or 2 micrograms of "mRNA NY-ESO-1 " (messenger RNA having the coding sequence of the protein NY-ESO-1 and diluted to 1 mg/ml with water), or 2 micrograms of the immunostimulating CpG oligonucleotide "CpG A", or with 0.5 micrograms of "R848", or with mRNA NY-ESO-1 mixed with protamine after both reagents have been diluted to 1 mg/ml with water (mRNA NY-ESO-1+Prot Water) or with mRNA NY-ESO-1 mixed with protamine after both reagents have been diluted to 1 mg/ml with Ringer Lactate (mRNA NY-ESO-1+Prot Ringer). Then, the supernatant of the cell culture was checked for the presence of interferon-alpha using an ELISA kit from Binder. The results show that, similar to RNA oligonucleotides, mRNA mixed with protamine induces a strong production of interferon-alpha, if both protamine and mRNA are diluted to 1 mg/ml using pure water (Fig. 5C). The presence of an isotonic salt concentration in protamine and mRNA before mixing (when both reagents are diluted to 1 mg/ml in Ringer Lactate) generates large particles which poorly stimulate interferon-alpha production by human PBMCs (Fig. 5C). The results also show that, similar to RNA oligonucleotides, RNA mixed with protamine induces a relatively poor production of TNF-alpha, if both protamine and mRNA are diluted to 1 mg/ml using pure water (Fig. 5D). The presence of an isotonic salt concentration in protamine and mRNA before mixing (when both reagents are diluted to 1 mg/ml in Ringer Lactate) generates large particles which strongly stimulate TNF-alpha production by human PBMCs (Fig. 5D).

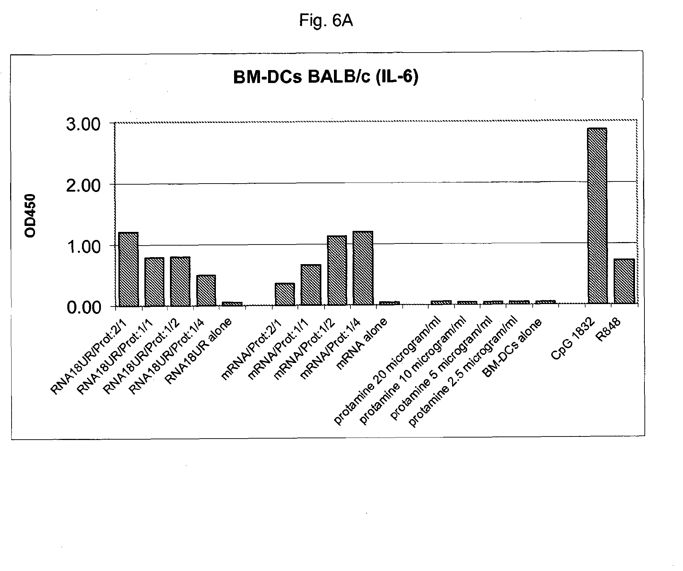

Fig. 6A: Bone marrow from BALB/c mice was collected and cells were cultured for one week in the presence of GM-CSF in order to obtain mouse Bone Marrow-Derived Dendritic Cells (BM-DCs). The cells were collected and distributed in a 96 well plate at a concentration of approx. 1 million cells per well (in 200 microliters of medium). RNA18UR, mRNA coding for NY-ESO-1 and protamine 1000 were diluted to 1 mg/ml. They were added alone to the cell cultures (as controls) or mixed at different ratios (as indicated) before being added to the cultures. 24 hours after incubation at 37°C, supernatants were collected and the Interleukine 6 content was evaluated using an ELISA (eBioscience). The results show that mouse BM-DCs are activated by protamine-RNA nanoparticles having a ratio of RNA/protamine 2:1 to 1:4. This result is independent of whether the RNA is an oligonucleotide or a long mRNA. As positive controls, the mouse TLR9-agonist CpG 1826 (10 micrograms per ml final concentration) and the TLR7-agonist R848 (2.5 micrograms per ml final concentration) were used.

Fig. 6B: Bone marrow from TLR7 knock-out mice was collected and cells were cultured for one week in the presence of GM-CSF in order to obtain mouse Bone Marrow-Derived Dendritic Cells (BM-DCs). The cells were collected and distributed in a 96 well plate at a concentration of approx. 1 million cells per well (in 200 microliters of medium). RNA18UR, mRNA coding for NY-ESO-1 and protamine 1000 were diluted to 1 mg/ml. They were added alone to the cell cultures (as controls) or mixed at different ratios (as indicated) before being added to the cultures. 24 hours after incubation at 37°C, supernatants were collected and the Interleukine 6 content was evaluated using an ELISA (eBioscience). The results show that mouse BM-DCs from TLR7 knock-out mice are not activated by protamine-RNA, demonstrating that TLR7 is involved in the recognition of protamine-RNA particles by immune cells (independent of whether RNA is an oligonucleotide or a long mRNA molecule). As a positive control, the mouse TLR9-agonist CpG 1832 (10 micrograms per ml final concentration) was used.

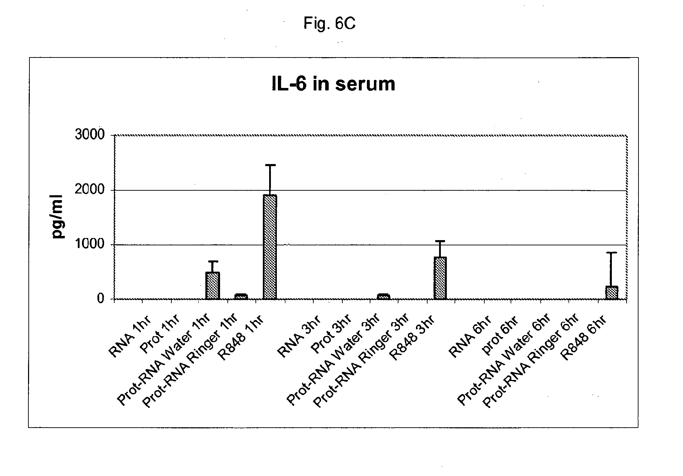

Fig. 6C: BALB/c mice were intravenously injected with 100 micrograms (50 micrograms protamine mixed with 50 micrograms RNA) of particles. Either small nanoparticles (Prot-RNA Water: protamine 5000 and RNA diluted to 1 mg/ml in pure water before mixing) or larger particles (Prot-RNA Ringer: protamine 5000 and RNA diluted to 1 mg/ml with Ringer Lactate before mixing) were injected and 1 hour, 3 hours or 6 hours later a drop of blood was collected from the mice (three mice for small and three mice for large particles as well as for the negative controls RNA alone and Protamine alone and for the positive control 10 micrograms of R848) The content of interleukine 6 in the serum was quantified by ELISA. It shows that only the injection of small nanoparticles induces a potent stimulation of the mouse immune system.

Fig. 7: The optimal ratio between protamine and RNA was tested using protamine 1000 diluted in water and RNA diluted to 1 mg/ml in water. This protocol results in nanoparticles (approx 500 nm) that can induce both IFN-alpha and TNF-alpha production. Thus, it allows us in one experiment to test the effect of protamine-RNA ratios on both IFN-alpha and TNF-alpha production. As before, 200 microliters of fresh human PBMCs at 5 million cells per ml were co-cultured with 4 micrograms of particles (2 micrograms of protamine and 2 micrograms of RNA) for 24 hours. Whether a long mRNA or a 21 residues oligonucleotide (Oligo 21: 5' GUUGCAUGGGCCUCAUAUACA 3' (SEQ ID NO: 2) is used the best immunostimulation is seen when protamine is at equal quantity to the RNA or when there is more protamine than RNA in grams.

Fig. 8: The optimal ratio between protamine and RNA as far as mRNA expression (translation after injection) was tested. Each BALB/c mouse was injected in the left ear with 10 micrograms of naked mRNA coding for luciferase. In the right ears the mice received a mixture of protamine and luciferase coding RNA where the ratios between the two products were as indicated. 18 hours later, the mice received luciferin intra-peritoneal (200 µl of 20 mg/ml luciferin solution) and the emission of light was monitored over 1 minute using a Xenogen IVIS device. The result shows that as opposed to what is seen for immunostimulation, for mRNA expression, there must be at least 4 times less protamine than RNA in order to allow RNA expression which is necessary, for example for mRNA-based vaccination.

Fig. 9. Data from literature as well as our own results (Fig. 10) indicate that in the RNA, U and G residues are immunostimulating. Moreover, it has been suggested in the prior art that double U or UG or GU pairs are important. We tested the immunostimulating capacity of an RNA oligonucleotide devoided of double U residues and of UG or GU doublets (RNA18 UP for U-poor). This 18-residue-oligonucleotide RNA18UP (5' AUCGCUAUCGAUCUCUAG 3' (SEQ ID NO: 3)) was diluted to 1 mg/ml in water and mixed with protamine 5000 diluted to 1 mg/ml in water in order to generate small nanoparticles. The particles were added to fresh human PBMCs at 5 million cells per ml and after 24 hours of culture, the content of IFN-alpha in the supernatant was measured by ELISA. As shown in Fig. 9, the RNA18UP mixed with protamine could induce IFN-alpha production similarly to the U-rich oligonucleotide RNA18UR. When RNA and protamine are diluted to 1 mg/ml in Ringer Lactate, as usual, low IFN-alpha production is triggered by the large nanoparticles/microparticles.

Fig. 10: The effect of the sequence of the RNA on immunostimulation was tested using protamine 1000 diluted in water and RNA diluted to 1 mg/ml in water. This protocol results in nanoparticles of approx. 500 nm in average that can induce both IFN-alpha and TNF-alpha production. Thus it allows us in one experiment to test the effect of protamine-RNA ratios on both IFN-alpha and TNF-alpha production. As before, 200 microliters of fresh human PBMCs at 5 million per ml were co-cultured with 4 micrograms of particles (2 micrograms of protamine and 2 micrograms of RNA) for 24 hours. Dodecamer RNA oligonucleotides of different sequences (A-rich 12m: GAAAAACAAGAA (SEQ ID NO: 4); U-rich 12m: GUUAUUCUUGUA (SEQ ID NO: 5); C-rich 12m: GCCACCCCCGCA (SEQ ID NO: 6), G-rich 12m: GGGAGGCGGAGA (SEQ ID NO: 7); Purine 12m: AGAGAGAGAGAG (SEQ ID NO: 8); Pyrimidine 12m: CUCUCUCUCUCU (SEQ ID NO: 9)) were used. Only the U-rich 12m oligonucleotide combined with protamine is strongly immunostimulating for both IFN-alpha and TNF-alpha production. The G-rich oligonucleotide, devoided of U residues, generates a detectable immunostimulating activity when mixed with protamine. The pyrimidine and purine oligonucleotides also show a detectable immunostimulating activity when mixed with protamine. These data confirmed previously published work demonstrating that mostly U-residues and to a lesser extend G-residues are immunostimulating entities within RNA.

Fig. 11: The effect of the length of the RNA on immunostimulation was tested using protamine 1000 diluted in water and RNA diluted to 1 mg/ml in water. This protocol results in nanoparticles of approx. 500 nm in average that can induce both IFN-alpha and TNF-alpha production. Thus, it allows one to test in one experiment the effect of protamine-RNA ratios on both IFN-alpha and TNF-alpha production. As before, 200 microliters of fresh human PBMCs at 5 million cells per ml were co-cultured with 4 micrograms of particles (2 micrograms of protamine and 2 micrograms of RNA) for 24 hours. Oligonucleotides of different length but always containing U-residues were tested (21merU: AGUGUUAUUCUUGUAUGGUUG (SEQ ID NO: 10); 18merU: AGUGUUAUUCUUGUAUGG (SEQ ID NO: 1); 16merU: GUGUUAUUCUUGUAUG (SEQ ID NO: 11); 14merU: GUUAUUCUUGUAUG (SEQ ID NO: 12); 12merU: GUUAUUCUUGUA (SEQ ID NO: 5); 10merU: GUUAUUCUUG (SEQ ID NO: 13)). The results show that although oligonucleotides of 10 residues are capable to provide immunostimulation when mixed with protamine, stimulation is increased by using oligonucleotides of 18 residues or more.

### Detailed Description of the Invention

As opposed to what is described in the prior art (see Scheel 2006, Fig. 1), the present inventors surprisingly discovered that nanoparticles of defined average size can be produced by mixing protamine and RNA. A preferred procedure for the preparation of such nanoparticles is to dilute protamine and RNA at concentrations of less than approx. 2 mg/ml, at best at 1 mg/ml or less using pure water or low salt solution (preferably less than 75 mM electrolytes, more preferred less than 25 mM electrolytes) and then mixing the two solutions.

It was furthermore discovered that depending on the salt concentration in protamine and or RNA solutions before mixing, not only the average size of the resulting particles is modified but also their immunostimulating capacity: For example, when protamine 5000 and RNA are diluted in pure water (or solutions containing less than 25 mM electrolytes) to 1 mg/ml or less (down to 0.25 mg/ml) and mixed together, the resulting particles of less than 450 nm diameter in average as described above, stimulate the highest production of interferon-alpha by PBMCs while the production of TNF-alpha is low.

It was also discovered that homogenous nanoparticles of slightly larger sizes (450 nm to 990 nm in average) are preferably obtained by diluting protamine 5000 and RNA to 1 mg/ml or less using 30 to 75 mM electrolyte solutions. Such particles can be obtained also in the presence of higher salts (diluting with 225 mM NaCl solution or isotonic solutions such as Ringer lactate), if the concentration of protamine and RNA is bellow 1 mg/ml before mixing.

Such larger nanoparticles induce both a strong production of IFN-alpha and of TNF-alpha by human PBMCs.

As shown here with protamine 5000 and RNA diluted to 1 mg/ml in Ringer Lactate and shown in the state of the art (Scheel 2005), the larger particles and aggregates obtained by mixing protamine and/or RNA in the presence of electrolytes induce a maximal production of TNF-alpha and a low production of IFN-alpha.

In order to generate highly immunostimulating nanoparticles the RNA molecules are preferably at least 10 residues in length and the mass ratio of protamine to RNA is preferably at least 0.5 (preferably not more than twice more RNA than protamine). According to a particularly preferred embodiment this ratio is 1 or higher (most preferred the same mass amount of protamine and RNA or more protamine than RNA is used).

The restriction concerning the ratio between protamine and RNA is the opposite of the state of the art: for mRNA vaccination, the ratio between protamine and mRNA should be less than 0.25 (at least four times more RNA than protamine) in order to guaranty mRNA expression (translation). The occupancy of protamine on RNA at mass ratios needed for producing immunostimulating nanoparticles (protamine/RNA > 0.5) probably prevents physically the translation of the RNA by ribosomes.

In the RNA molecule, contained in the nanoparticles for the present invention U doublets, GU or UG pairs are not required, since, for example, the U-poor oligonucleotide AUCGCUAUCGAUCUCUAG (SEQ ID NO: 3) when diluted to 1 mg/ml in pure water and mixed with protamine 5000 diluted to 1 mg/ml in pure water generates nanoparticles that induce a strong production of IFN-alpha.

The distinctive character of the subject matter of the present invention is shown by the fact that nanoparticles of defined average size (diameter) are provided, i.e. of about 50 to about 990 nm, preferably: less than 450 nm, in particular 50 to 450 nm for the smaller particles and, according to other preferred embodiments, 450 to 990 nm for larger nanoparticles, which are particularly suitable for the production of pharmaceutical compositions that can be administered i.v. or by other methods of administration.

A further benefit of the present invention is that through the specific method of production of the nanoparticles it is possible to control precisely the immune response: preferred production of IFN-alpha and low production of TNF-alpha using small particles (average size of about 50 to about 450nm) and production of both IFN-alpha and TNF-alpha by larger nanoparticles (average size of about 450 to about 990 nm). This is contrary to prior art approaches in which strong production of TNF-alpha and relatively low production of IFN-alpha using large particles/aggregates was obtained.

Based on the non-specific immunostimulation by protamine-RNA nanoparticles of defined size according to the present invention, they can be used to activate or strengthen the immunity against chronic diseases such as cancer or persistent virus infections.

Examples of cancers treatable with the nanoparticles and immunostimulating composition, respectively, according to the invention include malignant melanoma, all types of carcinoma (colon, renal cell, bladder, prostate, non-small cell and small cell lung carcinoma, etc.), lymphomas, sarcomas, blastomas, gliomas, etc.

Examples of infectious diseases treatable with the nanopartilces and immunostimulating composition, respectively, according to the invention include viral infectious diseases, such as AIDS (HIV), hepatitis A, B or C, herpes, herpes zoster (chicken-pox), German measles (rubella virus), yellow fever, dengue etc. flaviviruses, influenza viruses, hemorrhagic infectious diseases (Marburg or Ebola viruses), bacterial infectious diseases, such as Legionnaire's disease (*Legionella*), gastric ulcer (*Helicobacter*), cholera (*Vibrio*), infections by *E. coli*, *Staphylococci, Salmonella* or *Streptococci* (tetanus); infections by protozoan pathogens such as malaria, sleeping sickness, leishmaniasis; toxoplasmosis, i.e. infections by Plasmodium, Trypanosoma, Leishmania and Toxoplasma; or fungal infections, which are caused e.g. by *Cryptococcus neoformans, Histoplasma capsulatum, Coccidioides immitis, Blastomyces dermatitidis* or *Candida albicans*).

By switching the immunity, the immunostimulating nanoparticles and pharmaceutical composition, respectively, of the present invention may prove to be useful to treat allergies as well.

In one embodiment, the present invention provides nanoparticles, preferably in the form of an immunostimulatory composition, that comprise(s) a non-homopolymeric, non-modified, single stranded RNA.

The RNA is preferably an oligonucleotide of about 6 to 100 ribonucleotides or a messenger RNA (mRNA) of about 100 to 10,000 nucleotides.

Because manufacturing of oligonucleotides by chemical synthesis is easier in very large scales (kilograms) than the enzymatic synthesis of mRNA, oligonucleotides are the preferred RNA molecules for the purposes of the present invention.

Oligoribonucleotides can be also obtained from existing nucleic acid sources using isolation methods well-known to those ordinarily skilled in the art, but are preferably chemically synthesized (e.g. produced by oligonucleotide synthesis). Synthesized oligonucleotides are preferred because they are chemically well-defined and because of their purity and sequence homogeneity.

As used herein, the term "oligoribonucleotide" shall mean multiple ribonucleotides (i.e. a molecule comprising a ribose) linked to a phosphate group and to an organic base selected from the group consisting of cytosine (C), uracil (U) adenine (A) and guanine (G). An oligomer generally is defined to consist of a finite number of monomer units, which number ranges from a few to more than a hundred. In the context of the present invention, an oligoribonucleotide consists of about 6 to 100 ribonucleotides. Preferably, 12 to 40 and even more preferably 16 to 21.

The RNA of the present invention is preferably single-stranded and usually does not contain any chemical modifications to its subunits (e.g. on the base, or on the phosphate, or on the ribose residue). However, modifications that could help manufacturing or formulation or biological activities of the protamine-RNA nanoparticles described herein are also subject of the present invention. The 5' end of the RNA can be OH, monophosphate or triphosphate, the later allowing stimulation of the cytosolic RIG-1 and enhancing immunostimulation.

There is no specific ribonucleotide sequence requirement for an oligoribonucleotide molecule to be suitable for preparing an immunostimulatory nanoparticle composition according to the present invention. Preferably, however, the RNA contains at least 25% uridine residues.

Most preferably, the RNA is an oligonucleotide that has the following sequences (written 5' to 3'): RNA18UP: AUCGCUAUCGAUCUCUAG (SEQ ID NO: 3), 21merU: AGUGUUAUUCUUGUAUGGUUG (SEQ ID NO: 10); 18merU: AGUGUUAUUCUUGUAUGG (SEQ ID NO: 1); 16merU: GUGUUAUUCUUGUAUG (SEQ ID NO: 11); 14merU: GUUAUUCUUGUAUG (SEQ ID NO: 12); 12merU: GUUAUUCUUGUA (SEQ ID NO: 5); 10merU: GUUAUUCUUG (SEQ ID NO: 13); Oligo 21: GUUGCAUGGGCCUCAUAUACA (SEQ ID NO: 2).

The protamine of the present invention can be sulfated protamine or hydrochloride protamine. In a preferred embodiment, the protamine source used for the production of the nanoparticles and the "immunostimulatory protamine-RNA nanoparticle composition", respectively, is protamine 5000 which contains protamine at more than 10 mg/ml (5000 heparin-neutralizing units per ml) in an isotonic salt solution and which is diluted as set forth above.

Protamine is nontoxic, and is already used clinically, for example as anti-heparin treatment in surgery and as insulin stabilizer in new insulin formulations. Protamine was used previously to stabilize mRNAs in a mRNA vaccine preparation (EP 1818409), however it has been found that at the protamine : RNA mass ratio used for the instant invention (more than 1:2, at best 1:1, 2:1 or 4:1), the expression of mRNA is inhibited. Thus the method to use protamine for stabilizing mRNA according to the prior art is different than the one used to create immunostimulating homogeneous nanoparticles according to the present invention.

The immunostimulatory composition preferably has a protamine:RNA weight ratio from 8:1 to 1:2, more preferably from 4:1 to 1:2.

In a preferred embodiment, in the nanoparticles of the present invention, the weight ratio between the protamine and the oligoribonucleotide is about 1.

To further increase effectiveness, the immunostimulating compositions according to the invention can comprise one or more adjuvants, preferably to achieve a synergistic effect of immunostimulation. "Adjuvant" in this context encompasses any compound which promotes an immune response. Various mechanisms are possible in this respect, depending on the various types of adjuvants. For example, compounds which allow the maturation of the DC, e.g. lipopolysaccharides or CD40 ligand, form a first class of suitable adjuvants. Generally, any agent which influences the immune system of the type of a "danger signal" (LPS, GP96, dsRNA etc.) or cytokines, such as GM-CFS, can be used as an adjuvant which enables an immune response to be intensified and/or influenced in a controlled manner. CpG oligodeoxynucleotides can optionally also be used in this context, although their side effects which occur under certain circumstances, as explained above, are to be considered. Because of the presence of the immunostimulating agent according to the invention comprising RNA as the primary immunostimulant, however, only a relatively small amount of CpG DNA is necessary (compared with immunostimulation with only CpG DNA). Particularly preferred adjuvants are cytokines, such as monokines, lymphokines, interleukins or chemokines, e.g. IL-1, IL-2, IL-3, IL-4, IL-5, IL-6, IL-7, IL-8, IL-9, IL-10, IL-12, INFα, INF-γ, GM-CFS, LT-α, or growth factors, e.g. hGH. Further known adjuvants are aluminium hydroxide, Freund's adjuvant or oil such as Montanide^{®}, most preferred Montanide^{®} ISA51. Lipopeptides, such as Pam3Cys, are also particularly suitable for use as adjuvants in the immunostimulating composition of the present invention.

In a preferred embodiment, the immunostimulating composition according to the invention can also be used in conjunction with another therapeutic reagent. The immunostimulatory protamine/RNA composition of the present invention may on its own synergize with other treatments such as chemotherapeutic drugs for cancer patients or tri-therapy for HIV patients or chloroquine, a drug used against malaria infection and known to improve cross priming. Indeed, the immunostimulating composition alone is able to induce a nonspecific general activation of the immune system, which in turn helps the control of pathogens.

Many chemotherapy regimens (gemcitabine, etopophos, cis-platin, carbo-platin, etc.) or radiotherapy protocols do not severely affect the immune system. Thus, during radio/chemotherapy in cancer patients, immunomodulation can be used whereby the death of tumor cells can be accompanied by the induction of an immune response by immunostimulating compositions according to the present invention. Systemic (intra-venous or sub-cutaneous for example) as well as local (intra-tumor or intradermal for example) injections of an immunostimulatory protamine-RNA nanoparticle composition according to the present invention in patients under radio/chemotherapy may help the immune system to increase a response against the tumor. This immune response could also control tumor growth.

In a further embodiment, the pharmaceutical composition of the present invention takes the form of a vaccine preparation comprising the immunostimulatory protamine-RNA nanoparticles defined above and at least one antigen. An "antigen" is to be understood as meaning any structure which can cause the formation of antibodies and/or the activation of a cellular immune response. Examples of antigens are polypeptides, proteins, cells, cell extracts, carbohydrates/polysaccharides, polysaccharide conjugates, lipids, and glycolipids. These antigens may be tumor antigens or viral, bacterial, fungal and protozoological antigens or allergens. The antigen may be present in the vaccine according to the invention in the form of a hapten coupled to a suitable carrier. Suitable carriers are known to those ordinarily skilled in the art and include e.g. human serum albumin (HSA), polyethylene glycols (PEG). The hapten may be coupled to the carrier by processes well-known in the prior art, e.g. in the case of a polypeptide carrier via an amide bond to a Lys residue.

The formulation of antigen plus protamine-RNA nanoparticles may be formulated as an emulsion using mixing with an oil such as Montanide®.

In an alternative embodiment, the immunostimulating composition of the present invention may be used in genetic vaccination, wherein an immune response is stimulated by introduction into the organism or into the cell (in vitro electroporation followed by adoptive transfer or direct injection by needle-dependent or needle-less devices) a suitable nucleic acid molecule which codes for this antigen. This nucleic acid molecule may be a DNA or a mRNA. The "immunostimulatory protamine-RNA nanoparticle composition" can be injected systematically (intra-venous or sub-cutaneous) as well as locally at the site of DNA or mRNA delivery, thereby providing an immune environment (induction of cytokines and maturation of APCs) profitable to the induction of an immune response.

The vaccines according to the invention are suitable for the treatment of cancers. A tumor-specific antigen (TSA) or a nucleic acid which codes for such an antigen as well as part(s) of tumor antigens or nucleic acids which code for such part(s) may be used in this context. Specific examples of tumor antigens which can be used according to the invention include 707-AP, AFP, ART-4, BAGE, .beta.-catenin/m, Bcr-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R170I, HPV-E7, HSP70-2M, HAST-2, hTERT (or hTRT), iCE, KIAA0205, LAGE, LDLR/FUT, MAGE, MART-1/Melan-A, MC1R, myosin/m, MUC1, MUM-1, -2, - 3, NA88-A, NY-ESO-1, p190 minor bcr-abl, Pml/RAR.alpha., PRAME, PSA, PSM, RAGE, RU1 or RU2, SAGE, SART-1 or SART-3, TEL/AML1, TPI/m, TRP-1, TRP-2, TRP-2/INT2 and WT1.

The vaccine according to the invention may be furthermore employed against infectious diseases.

The vaccine according to the invention may be used in combination with chloroquine, a pharmaceutical compound that increases cross presentation and thus the induction of antigen-specific effector T-cells.

An example of the vaccine formulation to be used could be the full length NY-ESO-1 protein mixed with protamine-RNA nanoparticles and homogenized in Montanide^{®}. The emulsion may be administered subcutaneously, optionally in combination with a concomitant uptake of chloroquine by the treated individual.

The present invention is particularly suitable for use in inducing production, or increasing the level of, IFN-alpha. When added to human PBMC cells *in vitro,* the immunostimulatory composition is capable of inducing production of at least 500 pg/ml interferon IFN-alpha by 1 million human PBMCs cultivated 24 hours in 200 µl culture medium (RPMI plus 10% fetal calf serum).

Preferably, one form of the nanoparticles of the present invention (small nanoparticles), in particular in the form of an immunostimulatory composition, when co-incubated with human PBMC cells *in vitro,* for 24 hours is capable of inducing strong IFN-alpha production by 1 million human PBMCs (more than 500 pg/ml), but TNF-alpha production less than 10,000 pg/ml.

Another form of the nanoparticles of the present invention, preferably provided as an immunostimulatory composition, when co-incubated with human PBMC cells *in vitro,* for 24 hours is capable of inducing IFN-alpha production by 1 million human PBMCs (more than 100 pg/ml), and TNF-alpha production of more than 10,000 pg/ml.

In a further preferred embodiment, the nanoparticles of the present invention have an average size of not more than about 450 nm in diameter, preferably 50 to 450 nm in diameter (small nanoparticles).

In another preferred embodiment, the present invention provides larger nanoparticles of protamine and RNA having an average size of about 450 nm to about 990 nm in diameter.

The average "size" of the nanoparticles is generally the "design size" or intended size of the particles prepared according to an established process. Size may be a directly measured dimension, such as average or maximum diameter, or may be determined by an indirect assay such as a filtration screening assay. Direct measurement of particle size is typically carried out by dynamic light scattering. As minor variations in size arise during the manufacturing process, a variation up to 40% of the stated measurement is acceptable and considered to be within the stated size. Alternatively, microcarrier size may be determined by filtration screening assays. For example, a particle preparation is less than a stated size, if at least 97% of the particles pass through a "screen-type" filter of the stated size.

The compositions of the present invention can further be processed and can contain further active ingredients. For example, U.S. Pat. No. 7,018,657 discloses a method for preparing nanoparticulate formulations of pharmaceutical compositions comprising polynucleotides, pre-condensed with a polycationic peptide, such as protamine sulphate.

A further embodiment is an injectible formulation comprising the immunostimulating nanoparticles of the invention in combination with a pharmaceutically acceptable excipient such as Ringer Lactate.

The present invention further provides a method of immunostimulation, in particular for stimulating a host immune response in a subject, preferably a mammal, especially a human. An effective amount of a pharmaceutical composition according to the invention is administered, optionally in combination with another therapeutic treatment (for example, radiotherapy) or agent, such as a protein vaccine, a cancer chemotherapy agent, an additional immunomodulating agent, a pharmaceutical drug enhancing cross-priming such as chloroquine, an antiviral agent, anti-parasite agent or an anti-bacterial agent. Thus, the present invention also comprises the use of nanoparticles as defined herein for the preparation of a pharmaceutical composition or medicament for immunomodulation, in particular for stimulating the host immune response in a subject, preferably a mammal, especially a human.

Preferably, the additional immunomodulating agent is an anti-CTL-A4 or anti-regulatory T-cell reagents such as an anti-CD25 antibody or cyclophosphamide.

The at least one additional therapeutic agent may be administered simultaneously with the pharmaceutical composition, or the at least one additional therapeutic agent is administered sequentially with the pharmaceutical composition.

According to the method of the present invention, IFN-alpha level is increased by the administration of the immunostimulating composition of the invention.

In a further embodiment, the present invention provides an *ex vivo* method for stimulating host immune response in a mammal. In one embodiment, suitable immune cells are isolated from the mammal and are treated *in vitro* via administering to the isolated immune cells an effective amount of a pharmaceutical composition of the present invention, and the stimulated immune cells are re-introduced into the organism. Suitable immune cells for such *ex vivo* treatment include but are not limited to dendritic cells.

The method and composition of the present invention may be used to supplement interferon-α treatment, or to increase interferon-α in a subject.

The immunostimulating composition of the invention comprises, in addition to RNA and protamine, and other therapeutic or immunogenic agents, a pharmaceutically acceptable carrier and/or a pharmaceutically acceptable vehicle and/or pharmaceutically acceptable diluent. Appropriate routes for suitable formulation and preparation of the immunostimulating agent according to the invention and the vaccine are disclosed in Remington: "The Science and Practice of Pharmacy," 20th Edn., A.R. Gennaro, Editor, Mack Publishing Co., Easton, PA (2003). Possible carrier substances for parenteral administration are e.g. sterile water, Ringer, Ringer lactate, sterile sodium chloride solution, polyalkylene glycols, hydrogenated naphthalenes and, in particular, biocompatible lactide polymers, lactide/glycolide copolymers or polyoxyethylene/polyoxy- propylene copolymers. Immunostimulating agents and vaccines according to the invention can comprise filler substances or substances such as lactose, mannitol, substances for covalent linking of polymers, such as e.g. of polyethylene glycol, on to antigenic haptens, peptides or polypeptides according to the invention, complexing with metal ions or inclusion of materials in or on particular preparations of polymer compounds, such as e.g. polylactate, polyglycolic acid, hydrogel or to liposomes, microemulsions, micelles, unilamellar or multilamellar vesicles, erythrocyte fragments or spheroblasts. The particular embodiments of the immunostimulating agent and the vaccine are chosen according to the physical properties, for example in respect of solubility, stability, bioavailability or degradability. Controlled or constant release of the active drug (-like) components according to the invention in the vaccine or in the immunostimulating agent includes formulations based on lipophilic depots (e.g. fatty acids, waxes or oils). In the context of the present invention, coatings of immunostimulating substances and vaccine substances or vaccine compositions (all of them according to the invention) comprising such substances, namely coatings with polymers, are also disclosed (e.g. polyoxamers or polyoxamines). Immunostimulating or vaccine substances or compositions according to the invention can furthermore have protective coatings, e.g. protease inhibitors or permeability intensifiers. Preferred carriers are typically aqueous carrier materials, water for injection (WFI) or water buffered with phosphate, citrate, HEPES or acetate, or Ringer or Ringer Lactate etc. being used, and the pH is typically adjusted to 5.0 to 8.0, preferably 6.5 to 7.5. The carrier or the vehicle will additionally preferably comprise salt constituents, e.g. sodium chloride, potassium chloride or other components which render the solution e.g. isotonic. Furthermore, the carrier or the vehicle can contain, in addition to the abovementioned constituents, additional components, such as human serum albumin (HSA), polysorbate 80, sugars or amino acids.

The mode and method of administration and the dosage of the immunostimulating agent according to the invention and of the vaccine according to the invention depend on the nature of the disease to be treated, where appropriate the stage thereof, the antigen (in the case of the vaccine) and also the body weight, the age and the sex of the patient.

The immunostimulatory composition of the present invention may preferably be administered to the patient parenterally, e.g. intravenously, intraarterially, subcutaneously, intradermally or intramuscularly. It is also possible to administer the immunostimulating agent or the vaccine topically or orally. A further injection possibility is into a tumor tissue or tumor cavity (after the tumor is removed by surgery, e.g. in the case of brain tumors).

### Example

### Method for the preparation of an immunostimulatory protamine-RNA nanoparticle composition

An oligoribonucleotide of approximately 18 residues is synthesized and purified. The product is then lyophilized and resuspended at 1 mg/ml in pure water. Protamine Valeant^{®} 5000 is diluted 14 times in pure water to provide a solution of protamine at approximately 1 mg/ml in low salt. RNA and protamine are mixed together at equal volume. Mixing consists in several pipeting up and down or in vortexing. The formulation is left for a few minutes on the bench and can then be further diluted with injection solution (for example Ringer lactate) or mixed with the antigen and eventually Montanide^{®}. Should the protamine-RNA nanoparticle solution be too diluted, the nanoparticles can be recovered by centrifugation or freeze drying and be resuspended in the adequate volume of desired solution before being used for treatment formulation.

## Claims

1. A pharmaceutical composition containing nanoparticles of an average size of from 50 to 450 nm comprising protamine and RNA in combination with a pharmaceutically acceptable carrier, vehicle and/or diluent for use in immunostimulation.

2. The composition for use of claim 1 wherein the weight ratio of protamine to RNA is from 8:1 to 1:2, preferably from 4:1 to 1:2.

3. The composition for use of claim 1 or 2 wherein the RNA contains at least one U nucleotide and/or at least one G nucleotide.

4. The composition for use according to any one of claims 1 to 3 wherein the RNA is an oligonucleotide of from 6 to 100 nucleotides.

5. The composition for use of claim 3 or 4 wherein the RNA is an oligonucleotide having a sequence selected from the group consisting of SEQ ID NO: 1, 2, 3, 5,10,11,12 and 13.

6. The composition for use according to any one of claims 1 to 3 wherein the RNA is an mRNA of from 100 to 10,000 nucleotides.

7. The composition for use according to any one of claims 1 to 6 wherein the composition further contains at least one antigen.

8. The composition for use of claim 7 wherein the antigen is selected from the group consisting of 707-AP, AFP, ART-4, BAGE, beta.-catenin/m, Bcr-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R1701, HPV-E7, HSP70-2M, HAST-2, hTERT (or hTRT), iCE, KIAA0205, LAGE, LDLR/FUT, MAGE, MART-1 /Melan-A, MC1R, myosin/m, MUC1, MUM-1, -2, -3, NA88-A, NY-ESO-1, p190 minor bcr-abl, Pml/RAR.alpha., PRAME, PSA, PSM, RAGE, RU1 or RU2, SAGE, SART-1 or SART-3, TEL/AML1, TPI/m, TRP-1, TRP-2, TRP-2/INT2 and WT1.

9. The composition for use according to any one of claims 1 to 8 wherein the pharmaceutical composition further contains at least one adjuvant.

10. The composition for use of claim 9 wherein the adjuvant is an oil.

11. The composition for use according to any one of claims 1 to 10 wherein the composition is co-administered with a further immunomodulating agent selected from the group consisting of chemotherapeutic drugs, chloroquine, anti-CTLA-4 or anti-regulatory T-cell reagents.

12. A method for the preparation of nanoparticles of an average size of from 50 to 450 nm comprising the steps of:
(a) providing an aqueous solution of RNA
(1) at 1 mg/ml or more using a solution containing 0 to 75 mM electrolytes; or
(2) at less than 1 mg/ml using a solution containing 0 to 75 mM electrolytes;
(b) providing an aqueous solution of protamine
(1) at 1 mg/ml or more using a solution containing 0 to 75 mM electrolytes;or
(2) at less than 1 mg/ml using a solution containing 0 to 75 mM electrolytes; and
(c) combining the solutions obtained in steps (a1) and (b1) or mixing the solutions obtained in (a2) and (b2).

13. The method of claim 12 wherein step (a1) and/or (a2) comprises resuspending dried RNA in an aqueous solution containing 0 to 75 mM electrolytes.

14. The method of claim 12 or 13 wherein step (b1) and/or (b2) comprises diluting an aqueous isotonic stock solution containing 1000 to 5000 heparin-neutralizing units of protamine per ml with an aqueous solution containing 0 to 75 mM electrolytes.

15. The method of claim 13 or 14 comprising the steps of:
(a) providing an aqueous solution of RNA at less than 2 mg/ml by resuspending dried RNA in pure water;
(b) providing an aqueous solution of protamine at less than 2 mg/ml by diluting an aqueous isotonic stock solution containing 5000 heparin-neutralizing units of protamine per ml with pure water; and
(c) combining the solutions obtained in steps (a) and (b).

## Patentansprüche

1. Pharmazeutische Zusammensetzung, enthaltend Nanopartikel mit einer mittleren Größe von 50 bis 450 nm, umfassend Protamin und RNA, in Kombination mit einem pharmazeutisch verträglichen Träger, Vehikel und/oder Verdünnungsmittel zur Verwendung in der Immunstimulation.

2. Zusammensetzung zur Verwendung Anspruch 1, wobei das Gewichtsverhältnis von Protamin zu RNA 8:1 bis 1:2, vorzugsweise 4:1 bis 1:2, beträgt.

3. Zusammensetzung zur Verwendung nach Anspruch 1 oder 2, wobei die RNA mindestens ein U-Nukleotid und/oder mindestens ein G-Nukleotid enthält.

4. Zusammensetzung zur Verwendung nach einem der Ansprüche 1 bis 3, wobei die RNA ein Oligonukleotid mit 6 bis 100 Nukleotiden ist.

5. Zusammensetzung zur Verwendung nach Anspruch 3 oder 4, wobei die RNA ein Oligonukleotid ist, das eine Sequenz aufweist, die aus der Gruppe, bestehend aus SEQ ID NO: 1, 2, 3, 5, 10, 11, 12 und 13, ausgewählt ist.

6. Zusammensetzung zur Verwendung nach einem der Ansprüche 1 bis 3, wobei die RNA eine mRNA mit 100 bis 10.000 Nukleotiden ist.

7. Zusammensetzung zur Verwendung nach einem der Ansprüche 1 bis 6, wobei die Zusammensetzung außerdem mindestens ein Antigen enthält.

8. Zusammensetzung zur Verwendung nach Anspruch 7, wobei das Antigen aus der Gruppe, bestehend aus 707-AP, AFP, ART-4, BAGE, beta.-catenin/m, Bcr-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R170I, HPV-E7, HSP70-2M, HAST-2, hTERT (oder hTRT), iCE, KIAA0205, LAGE, LDLR/FUT, MAGE, MART-1/Melan-A, MC1R, myosin/m, MUC1, MUM-1, -2, -3, NA88-A, NY-ESO-1, p190 minor bcr-abl, Pml/RAR.alpha., PRAME, PSA, PSM, RAGE, RU1 oder RU2, SAGE, SART-1 oder SART-3, TEL/AML1, TPI/m, TRP-1, TRP-2, TRP-2/INT2 und WT1, ausgewählt ist.

9. Zusammensetzung zur Verwendung nach einem der Ansprüche 1 bis 8, wobei die pharmazeutische Zusammensetzung außerdem mindestens ein Adjuvanz enthält.

10. Zusammensetzung zur Verwendung nach Anspruch 9, wobei das Adjuvanz ein Öl ist.

11. Zusammensetzung zur Verwendung nach einem der Ansprüche 1 bis 10, wobei die Zusammensetzung zusammen mit einem weiteren immunmodulatorischen Mittel verabreicht wird, das aus der Gruppe, bestehend aus chemotherapeutischen Mitteln, Chloroquin und Anti-CTLA-4 oder anti-regulatorischen T-Zell-Reagenzien, ausgewählt ist.

12. Verfahren zur Herstellung von Nanopartikeln mit einer mittleren Größe von 50 bis 450 nm, umfassend die Schritte:
(a) Bereitstellen einer wässrigen Lösung von RNA
(1) mit 1 mg/ml oder mehr unter Verwendung einer Lösung, die 0 bis 75 mM Elektrolyte enthält; oder
(2) mit weniger als 1 mg/ml unter Verwendung einer Lösung, die 0 bis 75 mM Elektrolyte enthält;
(b) Bereitstellen einer wässrigen Lösung von Protamin
(1) mit 1 mg/ml oder mehr unter Verwendung einer Lösung, die 0 bis 75 mM Elektrolyte enthält; oder
(2) mit weniger als 1 mg/ml unter Verwendung einer Lösung, die 0 bis 75 mM Elektrolyte enthält; und
(c) Kombinieren der in den Schritten (a1) und (b1) erhaltenen Lösungen oder Mischen der in (a2) und (b2) erhaltenen Lösungen.

13. Verfahren nach Anspruch 12, wobei der Schritt (a1) und/oder (a2) das Resuspendieren getrockneter RNA in einer wässrigen Lösung, die 0 bis 75 mM Elektrolyte enthält, umfasst.

14. Verfahren nach Anspruch 12 oder 13, wobei der Schritt (b1) und/oder (b2) das Verdünnen einer wässrigen isotonischen Stammlösung, die 1000 bis 5000 Heparin-neutralisierende Einheiten Protamin pro ml enthält, mit einer wässrigen Lösung, die 0 bis 75 mM Elektrolyte enthält, umfasst.

15. Verfahren nach Anspruch 13 oder 14, umfassend die Schritte:
(a) Bereitstellen einer wässrigen Lösung von RNA mit weniger als 2 mg/ml durch Resuspendieren von getrockneter RNA in reinem Wasser;
(b) Bereitstellen einer wässrigen Lösung von Protamin mit weniger als 2 mg/ml durch Verdünnen einer wässrigen isotonischen Stammlösung, die 5000 Heparin-neutralisierende Einheiten Protamin pro ml enthält, mit reinem Wasser; und
(c) Kombinieren der in den Schritten (a) und (b) erhaltenen Lösungen.

## Revendications

1. Composition pharmaceutique contenant des nanoparticles présentant une taille moyenne comprise entre 50 nm et 450 nm comprenant de la protamine et de l'ARN en combinaison avec un porteur, un véhicule et/ou un diluant pharmaceutiquement acceptable à utiliser en immunostimulation.

2. Composition à utiliser selon la revendication 1, dans laquelle le rapport de poids entre la protamine et l'ARN est compris entre 8:1 et 1:2, de préférence entre 4:1 et 1:2.

3. Composition à utiliser selon la revendication 1 ou 2, dans laquelle l'ARN contient au moins un nucléotide U et/ou au moins un nucléotide G.

4. Composition à utiliser selon l'une quelconque des revendications 1 à 3, dans laquelle l'ARN est un oligonucléotide composé de 6 à 100 nucléotides.

5. Composition à utiliser selon la revendication 3 ou 4, dans laquelle l'ARN est un oligonucléotide comprenant une séquence sélectionnée dans le groupe comprenant les SEQ ID NO: 1, 2, 3, 5, 10, 11, 12 et 13.

6. Composition à utiliser selon l'une quelconque des revendications 1 à 3, dans laquelle l'ARN est un mARN composé de 100 à 10000 nucléotides.

7. Composition à utiliser selon l'une quelconque des revendications 1 à 6, dans laquelle la composition contient en outre au moins un antigène.

8. Composition à utiliser selon la revendication 7, dans laquelle l'antigène est sélectionné dans le groupe comprenant les éléments suivants: 707-AP, AFP, ART-4, BAGE, beta-caténine/m, Bcr-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R170I, HPV-E7, HSP70-2M, HAST-2, hTERT (ou hTRT), iCE, KIAA0205, LAGE, LDLR/FUT, MAGE, MART-1/Melan-A, MC1R, myosine/m, MUC1, MUM-1, -2, -3, NA88-A, NY-ESO-1, p190 mineur bcr-abl, Pml/RAR.alpha, PRAME, PSA, PSM, RAGE, RU1 ou RU2, SAGE, SART-1 ou SART-3, TEL/AML1, TPI/m, TRP-1, TRP-2, TRP-2/INT2 et WT1.

9. Composition à utiliser selon l'une quelconque des revendications 1 à 8, dans laquelle la composition pharmaceutique contient en outre au moins un adjuvant.

10. Composition à utiliser selon la revendication 9, dans laquelle l'adjuvant est une huile.

11. Composition à utiliser selon l'une quelconque des revendications 1 à 10, dans laquelle la composition est administrée conjointement avec un agent immunomodulant supplémentaire sélectionné dans le groupe comprenant des médicaments de chimiothérapie, la chloroquine, un anti-CTLA-4 ou des réactifs de cellules T anti-régulatoires.

12. Procédé de préparation de nanoparticules présentant une taille moyenne comprise entre 50 nm et 450 nm, comprenant les étapes suivantes:
(a) créer une solution aqueuse d'ARN:
(1) à 1 mg/ml ou plus en utilisant une solution contenant de 0 à 75 mM d'électrolytes; ou
(2) à moins de 1 mg/ml en utilisant une solution contenant de 0 à 75 mM d'électrolytes;
(b) créer une solution aqueuse de protamine:
(1) à 1 mg/ml ou plus en utilisant une solution contenant de 0 à 75 mM d'électrolytes; ou
(2) à moins de 1 mg/ml en utilisant une solution contenant de 0 à 75 mM d'électrolytes; et
(c) combiner les solutions obtenues aux étapes (a1) et (b1), ou mélanger les solutions obtenues aux étapes (a2) et (b2).

13. Procédé selon la revendication 12, dans lequel l'étape (a1) et/ou (a2) comprend la remise en suspension de l'ARN séché dans une solution aqueuse contenant de 0 à 75 mM d'électrolytes.

14. Procédé selon la revendication 12 ou 13, dans lequel l'étape (b1) et/ou (b2) comprend la dilution d'une solution aqueuse à base isotonique contenant de 1000 à 5000 unités de protamine de neutralisation de l'héparine par ml avec une solution aqueuse contenant de 0 à 75 mM d'électrolytes.

15. Procédé selon la revendication 13 ou 14, comprenant les étapes suivantes:
(a) créer une solution aqueuse d'ARN à moins de 2 mg/ml en remettant en suspension l'ARN séché dans de l'eau pure;
(b) créer une solution aqueuse de protamine à moins de 2 mg/ml en diluant une solution aqueuse à base isotonique contenant 5000 unités de protamine de neutralisation de l'héparine par ml avec de l'eau pure; et
(c) combiner les solutions obtenues aux étapes (a) et (b).
